# EUROPEAN PATENT APPLICATION

(11) **EP 4 464 351 A1**
(43) Date of publication of application: **20.11.2024**
(21) Application number: 23173328.8
(22) Date of filing: 15.05.2023
(51) Int. Cl.: A61M 11/00, A61M 15/00, A61M 16/00

(54) **INHALATION THERAPY DEVICE**

(71) Applicant: PARI Pharma GmbH, 82319 Starnberg (DE)
(72) Inventor: STOECKL, Carolin, 80331 München (DE); HOFFMANN, Tobias, 86938 Schondorf am Ammersee (DE); HEINE, Benjamin, 80687 München (DE); BUCHNER, Simon, 85241 Hebertshausen (DE); LACHMAYER, Stefanie, 86415 Mering (DE); FILIP, Eric, 81547 München (DE)
(74) Representative: Hoffmann Eitle

(57) **Abstract**

Inhalation therapy device comprising a housing (4) having a handle portion (12) for holding the inhalation therapy device during inhalation, a support surface (19) for placing the inhalation therapy device on a horizontal surface and a reservoir (2) for holding a liquid to be nebulized; a membrane unit (3) disposed in the housing and having a membrane (5) comprising a plurality of apertures (110), wherein the membrane (5) is disposed so that, when liquid is held in the reservoir (2), the liquid is supplied to a first side (5.1) of the membrane (5); an actuator (6) coupled to the membrane (5) for vibrating the membrane (5), whereby the liquid passes through the apertures (110) and an aerosol is generated at a second side (5.2) of the membrane (5) opposite to the first side (5.1); and a mouthpiece (27) having an outlet opening (8) defined by an outer edge (28) of the mouthpiece (27) for being put into the mouth of a user for inhaling the aerosol, wherein a center axis (CA₁) of the outlet opening (8) is non-parallel to the support surface (19) so that the outer edge (28) of the mouthpiece (27) is oriented in a direction towards the support surface (19) without interfering with a virtual extension (13) of the support surface (19).

## Description

### Technical Field

The present disclosure relates to an inhalation therapy device for generating and delivering an aerosol to a user.

### Background

Inhalation therapy devices, also known as (inhalation) nebulizers or aerosol delivery devices, are widely used to deliver therapeutically effective amounts of pharmaceuticals, such as drugs and vaccines, in an aerosol form to users through their respiratory system. Inhalation therapy devices can also be used for diagnostic purposes using radioisotopes for lung challenge tests. For therapy, aerosol inhalation is the preferred root of administration for some pharmaceuticals, which can be intended for treatment of systemic or respiratory diseases.

To achieve the intended treatment, aerosol particles must be deposited in specific parts of the respiratory tract of the user's body, such as the lung. Different size particles tend to deposit in different parts of the respiratory system. It is commonly known in this field that particles having a MMD (mass median diameter) of less than 5µm such as between 1µm and 5µm or between 1µm and 4µm are required to allow deposition in the intended part of the respiratory system.

The aerosols for therapeutic purposes are generated and delivered to a desired location within the user's body, especially its respiratory tract, with the inhalation therapy device. To do so, a fluid, particularly a liquid, i.e., a medicament, a drug, a vaccine, etc., to be aerosolised or nebulised is supplied to a reservoir for holding the fluid or liquid in the inhalation therapy device. For aerosolising or nebulizing the liquid in the reservoir, inhalation therapy devices may, for example, comprise a membrane unit.

Such membrane units comprise a membrane having a plurality of apertures and an actuator directly or indirectly, via a support plate supporting the membrane, coupled to the membrane for vibrating the same.

The fluid or liquid may be in contact with a first side of the membrane via gravitational force. The liquid or fluid (i.e., the medicament) passes through the apertures from the first side of the membrane and an aerosol is generated at a second side of the membrane opposite to the first side of the membrane upon vibrating the membrane.

The aerosolised or nebulised liquid or fluid, i.e., the aerosol, inside the inhalation therapy device is then supplied to the user's respiratory system, within the bounds of an inhalation therapy upon inhalation of the user through the inhalation therapy device. Meaning, upon the application of a suction force by the user's breathing, especially his or her inhalation, through the inhalation therapy device the aerosol is at least partially transported (by suction force) from the interior of the inhalation therapy device to the patent's respiratory tract.

An example for a conventional inhalation therapy device having such a configuration is, for example, derivable from DE 199 53 317 C1. The membrane unit of the disclosed inhalation therapy device comprises a cylindrical liquid reservoir container, which is delimited at one end face by a membrane having the shape of a circular disc. A liquid disposed in the reservoir contacts the side of the membrane facing the reservoir.

DE 199 53 317 C1 further discloses a membrane connected (welded) to a substrate (support plate) and an oscillating generator, for example, a piezo crystal, which surrounds the membrane in a circular manner and is connected (glued) to the substrate, such that the membrane can be caused to oscillate by means of the oscillating generator and an electric drive circuit. Thereby, the liquid applied to the membrane on the first side of the membrane is conveyed through the apertures in the oscillating membrane to the second side of the membrane opposite to said first side of the membrane to be emitted into a chamber as an aerosol. The disclosed inhalation therapy device further embodies a mouthpiece or mask connected to an outlet of the chamber and having an exhalation valve. Upon exhalation of a user, the exhaled flow will, thus, not or only to a limited extend enter the chamber and rather be expelled through the exhalation valve into the environment of the device.

In general, the inhalation therapy devices on the market are held by hand while the user is sitting. The treatment time, i.e. the time periods during which the user actually inhales and exhales while aerosol is deliver it during inhalation, may be a significant time period. Although there is an aim and to keep the treatment time short, use of the inhalation therapy device, particularly holding the inhalation therapy device, may be perceived inconvenient.

In addition, the user may need to fill liquid into the reservoir before starting the treatment. For this purpose, it would be convenient to place the inhalation therapy device on a horizontal surface such as a table. Yet, some devices on the market tend to be relatively large in height and therefore tend to tip over while being filled. Beside the risk of the liquid leaking from the reservoir and being spilt over the horizontal surface, the mouthpiece or a mask may come into contact with the horizontal surface with the risk of being contaminated.

Hence, there remains a need for a simple and effective inhalation therapy device that is convenient to use.

### Summary

In view of the aforesaid, it is an object of the present disclosure to establish an inhalation therapy device that is improved in its ergonomics and convenient to use.

This object is solved by means of an inhalation therapy device according to independent claim 1. Distinct embodiments are derivable from the dependent claims and the following description.

According to a first aspect, the inhalation therapy device comprises a housing, a membrane unit and a mouthpiece. The housing has a handle portion for holding the inhalation therapy device during inhalation, a support surface for placing the inhalation therapy device on a horizontal surface and a reservoir for holding a liquid to be nebulized.

The handle portion is that portion of the housing, which is to be gripped by a user when using the inhalation therapy device. Optionally, the handle portion is gripped by one hand only for holding the inhalation therapy device during inhalation.

The support surface may, in one example, part of the handle portion, i.e. located in the handle portion. The main purpose of the support surface is to enable placing of the inhalation therapy device on a horizontal surface when not in use or during preparation for use, such as filling liquid into the reservoir. Consequently, the support surface may also be referred to as stand. The horizontal surface may for example be a table. The housing may comprise feet and the support surface may be a level surface formed by the respective stand surfaces of the feet.

The reservoir may be accommodated or formed in the housing or a housing body of the housing. The reservoir may be an integrated portion of the housing. In one example, the reservoir and the housing may be integrated as one - piece, e.g. in an injection moulding process. In another example, the reservoir and the housing body may be separately manufactured and integrated by being fixed, such as non-detachably fixed, to each other. Alternatively, the reservoir may be an ampoule exchangeably inserted into and opened in the housing. The usual amount of liquid to be contained in the reservoir is 1 ml to 12 ml or 1ml to 8 ml, however, 1ml to 6 ml are also possible.

The inhalation therapy device further comprises a membrane unit disposed in the housing. Membrane units are in the art also often referred to as "aerosol generator" or "head". The membrane unit has a membrane and an actuator.

The membrane comprises a plurality of apertures (through holes), wherein the membrane is disposed so that, when liquid is held in the reservoir, the liquid is supplied to a first side of the membrane. The membrane may be a circular disk having an active area in its center, wherein the apertures are formed in the active area. The membrane may be manufactured from metal, for example from stainless steel, or other suitable materials. The membrane is disposed or accommodated in the housing body. The liquid in the reservoir is suppliable to a first side of the membrane by gravitational force. The reservoir may for this purpose have an outlet opening surrounded by a sealing lip abutting the surface of the membrane on the first side an surrounding the active area.

The actuator is coupled to the membrane for vibrating the membrane. The actuator may be a piezoelectric element, such as a piezoelectric ring. The actuator may be directly coupled or attached to the membrane, that is either to the first side of the membrane or to a second side of the membrane opposite to the first side. Alternatively, the membrane may be coupled or attached to a support plate, such as an annular support plate and the actuator may be directly coupled or attached to the support plate. Upon activating or operating the actuator, the membrane is vibrated. Upon vibration of the membrane, the liquid passes through said apertures and an aerosol is generated at the second side of the membrane opposite to the first side.

The inhalation therapy device further comprises a mouthpiece. The mouthpiece has an outlet opening (in the following also often referred to the first opening) defined by an outer edge of the mouthpiece for being put into the mouth of a user for inhaling the aerosol. Under certain circumstances, a face mask covering nose and mouth may be attached to the mouthpiece. Yet, the mouthpiece is already configured to be used as a mouthpiece without the need of any additional members to be attached to the mouthpiece. Further, the mouthpiece may be an integrated part of the housing (the housing of the mouthpiece may be one piece). Alternatively, the housing may comprise a boss defining an opening at a mouthpiece as a separate member may be attached to the boss.

For convenient use of the inhalation therapy device, a center axis of the outlet opening is non-parallel to the support surface so that the outer edge of the mouthpiece is oriented in a direction towards the support surface. In other words, the outer edge of the mouthpiece faces a virtual extension of the support surface on a mouthpiece side. The support surface is a surface defined by the inhalation therapy device itself will particularly the housing and corresponds to a surface of the housing parallel to the horizontal surface on which the housing is placed. A virtual extension of this support surface is to be considered as an increase of this surface in all directions. When in use, the mouthpiece or at the center axis of the mouthpiece will be substantially horizontal ± 20° and the support surface may be inclined relative to the horizontal. Accordingly, the inhalation therapy device may easily be gripped and conveniently be held by one hand of a user.

Because the outer edge of the mouthpiece is however configured so as to not interfere with the virtual extension of the support surface, the outer edge of the mouthpiece does not come in contact with the horizontal surface on which the inhalation therapy device is placed by means of its support surface. Accordingly continuation of the outer edge of the mouthpiece can effectively be avoided.

According to a second aspect, the minimum distance between the virtual extension of the support surface and the outer edge of the mouthpiece in a direction perpendicular to the virtual extension of the support surface is more than 3 mm and less than 15 mm, preferred more than 4 mm and less than 12 mm.

This range particularly realizes that the outer edge of the mouthpiece is sufficiently distanced from the horizontal surface on which the inhalation therapy device is placed for hygienic purposes and maintaining the height of the inhalation therapy device when placed on the horizontal surface relatively low to prevent tipping over.

According to a third aspect, the center axis of the outlet opening (first opening) intersects the virtual extension of the support surface on a mouthpiece side with respect to the support surface and an angle α₁ between the virtual extension of the support surface and the center axis of the outlet opening is, in side view (longitudinal cross-sectional view including the center axis), an acute angle of less than 75°, optionally between 10° and 70°, optionally between 15° and 60°, optionally between 20° and 50°.

This particular angle between the center axis of the outlet opening and the support surface assists in achieving that the inhalation therapy device may easily be gripped and conveniently be held by one hand of a user during use, in which the center axis of the outlet opening is substantially horizontal ± 20°.

According to a fourth aspect, the reservoir has a filling opening for receiving the liquid and a lid for closing the filling opening. In this context, the filling opening is to be understood as that opening defined by a circumferential rim of the reservoir distanced from the outer edge (end face) of the circumferential rim facing the lid by around 10 mm (±2mm).

The lid may be unscrewed from the reservoir and the to be administered liquid may be poured into the reservoir via the filling opening. When the inhalation therapy device 1 is placed with its support surface on a horizontal surface such as a table, a plane of the filling opening will be angled towards the mouthpiece relative to the horizontal surface. Yet, pouring liquid into the reservoir via the filling opening is still enabled without liquid flowing out of the reservoir. More than that, due to this configuration, the filling opening is tilted relative to the support surface and, hence, the horizontal surface making it more ergonomic to fill. Similar to how you tilt a glass when pouring. Subsequently, the lid may again be screwed to the reservoir to seal the liquid. An angle α₂ between the support surface and a center axis of the filling opening is, in side view (longitudinal cross-sectional view including the center axis), an obtuse angle of more than 90°±5°, optionally between 100° and 160° and preferred between 110 and 150°.

According to this aspect, filling of the reservoir when the inhalation therapy device is placed with the support surface on e.g. a table is simplified at the same time realizing an upright position of the reservoir during use for good delivery of the liquid to the first side of the membrane, particularly by gravitational force.

According to a fifth aspect, and angle α₃ between the center axis of the filling opening and the center axis of the mouthpiece is, in side view (longitudinal cross-sectional view including the center axes), is between 70° and 110° and preferred 80° and 100° and even more preferred around 90°±5°.

This particular angle assists in achieving that the reservoir assumes an upright position during use for good delivery of the liquid to the first side of the membrane, particularly by gravitational force. To even further promote the delivery of the liquid to the first side of the membrane, the reservoir may taper towards the first side of the membrane and particularly towards a reservoir outlet opening surrounding the active area of the membrane.

According to a sixth aspect, an angle α₄ between the support surface and the membrane is, in side view (longitudinal cross-sectional view perpendicular to the support surface and the plane defined by the membrane), is an obtuse angle of more than 90°±5°, optionally between 100° and 160° and more optionally between 110° and 150°. Thus, the membrane may be oriented relative to the support surface in the same manner as the center axis of the filling opening.

This particular angle also assists an upright position of the inhalation therapy device and particularly a substantially vertical ± 20° orientation of the membrane during use and, hence, good alignment of the nebulization direction with the air flow.

According to a further aspect, the center axis (plane normal of the membrane, particularly its active area) of the membrane, particularly its active area, is oriented so as to not intersect with the housing but only the cross-sectional area defined by the outlet opening, i.e. the outer edge.

According to a seventh aspect, the handle portion comprises the support surface and a curved surface opposite to the support surface wherein the curved surface, in side view (longitudinal central cross section of the housing), defines a ridge line. In this context, a ridge line is to be understood as a line formed along the highest points of the upper surface in side view (longitudinal central cross-sectional view).

According to this aspect, the housing in the handle portion is ergonomically shaped providing for a convenient gripping of the inhalation therapy device during use.

According to an eighth aspect, an angle α₆ between the ridge line and the support surface is, in side view, less than 20°, optionally between 5° and 15°.

This particular angle assists in ergonomically configuring the handle portion of the housing.

According to a ninth aspect, the inhalation therapy device further comprises at least one control button and/or at least one light indicator located on the curved surface, optionally centered on the ridge line.

These features particularly enhance the possibility of holding and operating the inhalation therapy device by only one hand in a comfortable and relaxed position.

According to a tenth aspect, the housing comprises a nebulizer housing and controller housing. The nebulizer housing has or defines the reservoir and comprises mouthpiece. Further, the mouthpiece and the reservoir may be an integrated (one - piece) part of the nebulizer housing. The nebulizer housing further accommodates the membrane unit. Further, the nebulizer housing may comprise a main body and a door hinged to the main body. Thus, the door may be moved relative to the main body and the membrane unit may be interchangeable. When placing the membrane unit e.g. inside the flow path, a cavity is formed between the membrane unit and the second opening (see later) which would be hard to clean. By configuring the nebulizer housing to comprise the main body and the hinged door, the membrane unit may be removed, and the cavity is accessible for being cleaned.

The controller housing accommodates a controller, such as a circuit board including various electronic and/or electric components. The controller is configured to control the actuator of the membrane unit. Further, the controller housing has the support surface and handle portion as well as, the optional curved surface, the operating button, and the light indicator. Moreover, the nebulizer housing is detachable from the controller housing.

Thus, the nebulizer housing may be configured as a disposable component that is exchanged from time to time, whereas the controller housing is a durable part of the inhalation therapy device. In addition, the nebulizer housing may be cleaned, disinfected, or sterilized separately from the controller housing or the controller housing may even not require disinfection or sterilization. If the nebulizer housing can be opened as discussed above, the membrane unit may be interchanged, which further simplifies the cleaning process.

According to an eleventh aspect, one of the nebulizer housing and the controller housing comprises a circumferential rim, which may define a recess, and the other of the nebulizer housing and the controller housing comprises a boss, wherein the circumferential rim or recess and the boss are engaged.

As a result, assembly of the inhalation therapy device for example after cleaning and alignment of the controller housing at nebulizer housing are simplified.

According to a twelfth aspect, the nebulizer housing defines a flow path from at least one inlet opening (also referred to as second opening in this disclosure) of the nebulizer housing to the outlet opening (first opening) of the mouthpiece, wherein a flow is generatable in the flow path upon inhalation of a user from the inlet opening passed the membrane unit to the outlet opening for entraining and delivering the generated aerosol and/or upon exhalation of a user from the outlet opening passed the membrane unit to the inlet opening.

As a result, a user has to inhale and exhale through the flow path of the inhalation therapy device without the need of removing the mouthpiece from the most during treatment, which further enhances convenience of the user and enables to monitor treatment quality more closely.

According to a thirteenth aspect, the controller housing comprises a socket and the membrane unit comprises a plug releasably plugged into the socket in a plugging direction, wherein an obtuse angle α₅ between the support surface and the plugging direction is, in side view (cross-sectional longitudinal view perpendicular to the support surface and including the plugging direction), more than 90°±5°, optionally between 100° and 160° and more optionally between 110° and 150°.

This particular angle enables that the nebulizer housing and the controller housing accommodating the membrane unit may conveniently be assembled when the controller housing is with its support surface placed on a horizontal surface such as a table.

According to a fourteenth aspect, the aerosol therapy device further comprises at least one battery or rechargeable battery accommodated in the housing, the battery being disposed in proximity of the support surface and optionally extending with its longitudinal extension along the support surface. In this context, a battery or rechargeable battery is to be understood as any container consisting of one or more cells, in which chemical energy is converted into electricity and used as a source of power. Further, along the support surface does not necessarily mean extending parallel to the support surface. Yet, the longitudinal direction of the battery should be within 0° and 20° and preferred within 0° and 15° to the support surface.

According to this aspect, the center of gravity of the inhalation therapy device, particularly the controller housing is relatively low providing for a good and the safe stand of the inhalation therapy device on a horizontal surface minimizes the risk of tipping over.

### Brief Description of the Drawings

Hereinafter, non-limiting examples of the disclosure are explained with reference to the drawings, in which:
Figure 1 shows an inhalation therapy device according to the present disclosure in an isometric view.
Figure 2 shows a side view of the inhalation therapy device of figure 1 placed on a horizontal surface.
Figure 3 shows a longitudinal cross section of the inhalation therapy device in figure 1.
Figure 4 shows an explosive view of the inhalation therapy device of figure 1.
Figure 5 shows a side view of an inhalation therapy device in a use position.

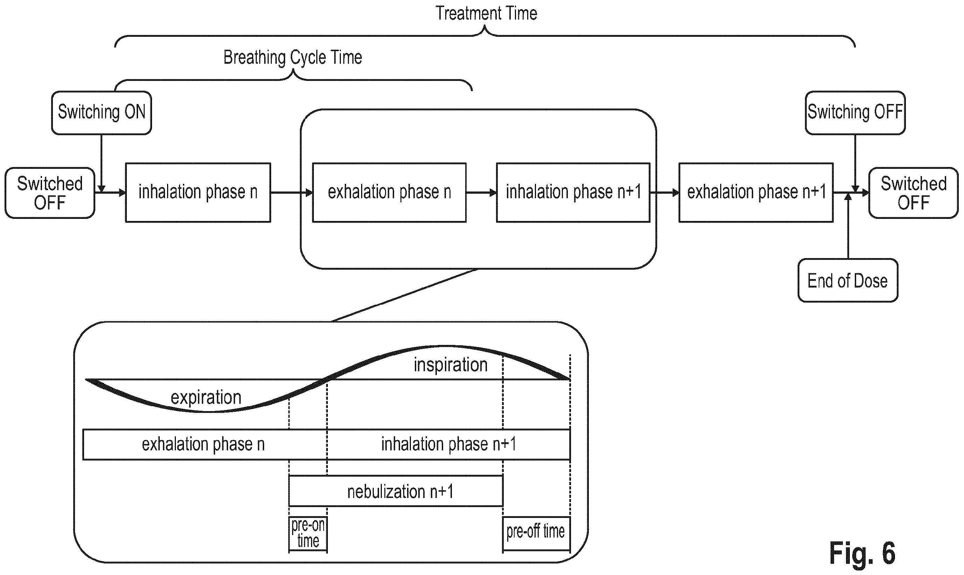

Figure 6 schematically shows the control of an inhalation therapy device of figures 1 to 5.
Figure 7 shows a partial cross section of the active area of the vibratable membrane according to an embodiment.
Figure 8 shows a partial cross section of the active area of the vibratable membrane according to another embodiment.

### Detailed Description

An inhalation therapy device of the present disclosure will now be described with reference to the accompanying drawings.

It is to be understood that such inhalation therapy devices 1 are oftentimes also called "aerosol delivery devices" or "nebulizers", for example for a therapeutic use in/via a user's respiratory system.

Further, the term "user", which has been used throughout the disclosure may be a patient.

In case that specific angles are defined in the present disclosure, positive angles are always taken between respective legs or planes in a counterclockwise direction.

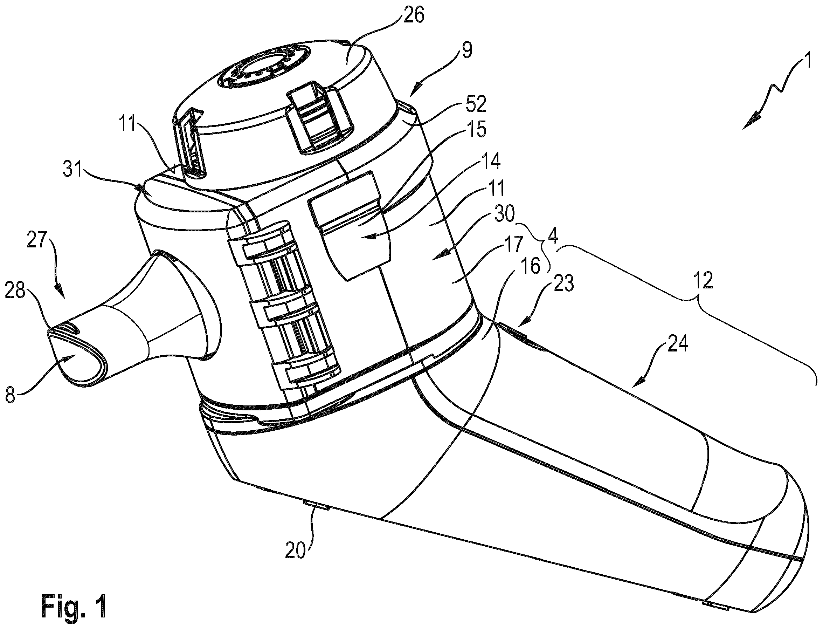

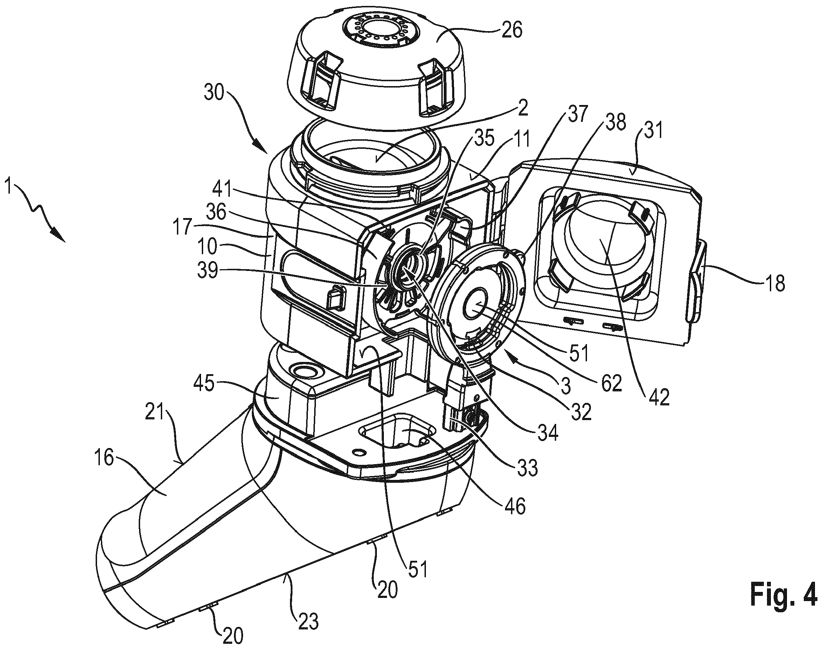

Fig. 1 shows an exemplary isometric view of the inhalation therapy device 1. The inhalation therapy device comprises a housing body 4. The housing body 4 may be split into two main components 16, 17 as best visible from figure 4.

In particular, the housing body 4 comprises a controller housing (which may also be referred to as holding portion or handle portion) 16 and a nebulizer housing 17 (which may also be referred to as aerosolization portion). Yet, the present disclosure is not limited to such a "split" configuration: it may also be possible to form the controller housing 16 and the nebulizer housing 17 integrally.

The controller housing 16, in the embodiment, also functions as or comprises a handle portion 12 during therapy in that the user grips the handle portion and thereby holds the inhalation therapy device 1.

The controller housing 16 further comprises a support surface 19 at its lower side, optionally in the handle portion 12, for placing the inhalation therapy device 1 on a horizontal surface such as a table. The support surface 19 may be defined by a plurality of feet 20 disposed on the bottom surface/lower surface of the controller housing 16. To put it differently, the housing body 4, particularly the controller housing 16, comprises the feet 20 and the support surface 19 is a level surface formed by the respective stand surfaces of the feet 20.

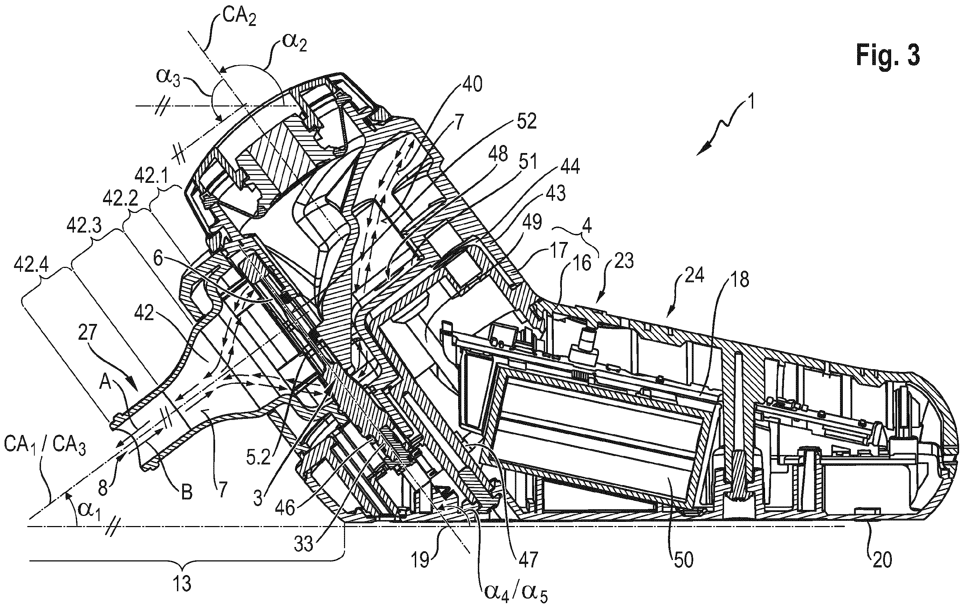

The nebulizer housing 17 has or defines a reservoir 2 for holding a liquid to be aerosolized (see figure 3). The reservoir 2 has a filling opening 25 for receiving the liquid and a lid 26 for closing the filling opening 25 (see figure 4). An angle α₂ between the support surface 19 and a center axis CA₂ of the filling opening 25 is, in side view (longitudinal cross-sectional view), an obtuse angle of more than 90°±5°, optionally between 100° and 160° and more optionally between 110° und 150°. An angle α₃ between the center axis CA₂ of the filling opening 25 and the center axis CA₁ of the mouthpiece is, in side view (longitudinal cross-sectional view), between 70° and 110° and optionally 80° and 100° and more optionally 90° ±5°.

The nebulizer housing 17 further defines a flow path 7 having a first opening (outlet opening) 8 to the outside of the housing body 4, particularly the nebulizer housing 17, at one end and at least one second opening (inlet opening) 9 to the outside of the housing body 4, particularly the nebulizer housing 17, at another end, so that a flow is generatable in a first flow direction A from the second opening(-s) 9 to the first opening 8 in the flow path 7 upon inhalation of a user at the first opening 8 for entraining and delivering the generated aerosol, and in a second flow direction B from the first opening 8 to the second opening(-s) 9 in the flow path 7 upon exhalation of a user into the first opening 8.

In this connection, the above-mentioned flow in the first flow direction A from the second opening(-s) 9 to the first opening 8 may also be understood as an "inhalation flow". Since the exhalation of the user shall also be performed through the flow path 7 of the inhalation therapy device 1, a second flow direction B from the first opening 8 to the second opening(-s) 9 in the flow path 7 upon exhalation of the user at the first opening 8 is understood as an "exhalation flow".

The first opening 8 is in the present example part of a mouthpiece 27 and defined by an outer edge 28 of the mouthpiece 27. The mouthpiece 27 is configured for being put into the mouth of a user for inhaling the generated aerosol.

The mouthpiece 27 or particularly its first opening 8 defines a center axis CA₁. The center axis CA₁ is non-parallel to the support surface 19. The center axis CA₁ of the first opening 8 intersects the virtual extension 13 of the support surface 19 on a mouthpiece 27 side with respect to the support surface 19 and an angle α₁ between the virtual extension of the support surface 19 and the center axis CA₁ of the first opening 8 is, in side view (longitudinal cross-sectional view) (figure 3), an acute angle of less than 75°, optionally between 10° and 70°, optionally between 15° and 60°, optionally between 20° and 50°.

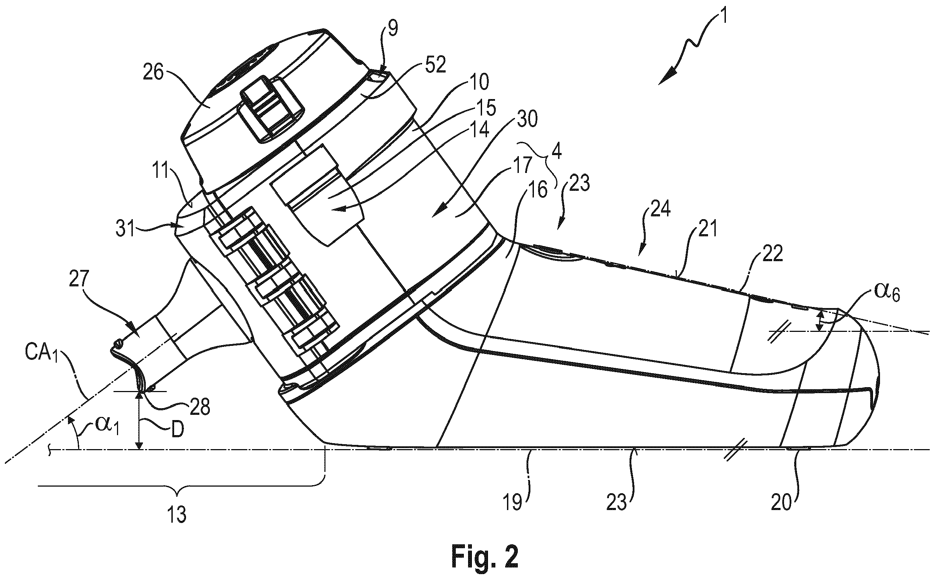

Accordingly, the outer edge 28 of the mouthpiece 27 is oriented in a direction towards the support surface 19. Yet, as will be apparent from figures 2 and 3, the outer edge 28 does not interfere with the virtual extension 13 of the support surface 19 and, hence, if placed on a horizontal surface such as a table, does not come into contact therewith. The minimum distance D between the virtual extension 13 of the support surface 19 and the outer edge 28 of the mouthpiece 27 in a direction perpendicular to the virtual extension 13 of the support surface 19 is more than 3 mm and optionally more than 4mm and less than 15 mm and optionally less than 12 mm.

Furthermore, a mixing chamber 42 is formed between the first opening 8 and the membrane unit 3, particularly the membrane 5, i.e. downstream of the membrane unit 3in the first flow direction A. The mixing chamber 42 is a volume defined between the first opening 8 and the membrane unit 3. Considering figure 3, the mixing chamber 42 as a first portion 42.1 with a constant cross-sectional area adjacent the membrane unit 3. In a direction towards the first opening 8, the mixing chamber 42 then abruptly decreases in cross-sectional area being substantially constant in cross-sectional area in a second portion 42.2. In a third portion, continuously tapering portion, 42.3, the mixing chamber 42 continuously tapers towards the first opening 8. The mixing chamber 42 are downstream of the third portion 42.3 a fourth portion 42.4 again with constant cross-sectional area ending in the first opening 8. Yet, it is also conceivable and may even be preferred that the portion 42.2 is omitted.

Moreover, the nebulizer housing 17 comprises a circumferential wall 10, a top wall 11 also comprising the filling opening 25 and a bottom wall 51. A transition between the top wall 11 and the circumferential wall 10 may be chamfered 52. The mouthpiece 27 is connected to the circumferential wall 10.

The second opening(-s) 9 is according to an example located adjacent the top wall 11 and in the present embodiment in the chamfer 52. Accordingly, second opening(-s) 9 are positioned most distant from the handle portion 12 so that the risk of blocking the second opening(-s) 9 by the hand or finger of a user can be minimized. The second opening(-s) 9 serve(-s) as the sole opening(-s) to the flow path 7 via which air may enter the flow path 7 upon inhalation of a user. In addition, the second opening(-s) 9 provide for the smallest cross-sectional area throughout the flow path 7 so that the second opening(-s) 9 exclusively govern the first flow resistance, i.e. inhalation flow resistance.

The nebulizer housing 17 may further comprise a third opening 14 or a plurality of third openings 14, which may also be referred to as exhalation opening(-s). The third opening(-s) 14 may be provided in the circumferential wall 10. The third opening(-s) 14 is (are each) covered by a check valve 15, particularly a flap valve (see figures 1 and 2) covering the third opening(-s) 14 on the outside of the nebulizer housing 17. The check valve 15 is configured to restrict a flow through the third opening(-s) 14 in the first flow direction A. That is, the check valve 15 prohibits any flow through the third opening(-s) 14 of the flow path 7 upon inhalation. However, the check valve 15 is also configured to enable a flow through the third opening(-s) 14 in the second flow direction B from the first opening 8 to the second opening(-s) 9 for enabling at least a partial expel of air through the third opening(-s) 14 upon exhalation. The third opening(-s) 14 to the outside of the housing body 4/nebulizer housing 17 is (are) provided in the flow path 7 between the first opening 8 and the second opening(-s) 9, particularly between a later described membrane unit 3 and the second opening(-s) 9, i.e. in the present embodiment in a storage chamber 40 (described later). To put it differently the third opening(-s) 14 is (are) arranged upstream of the membrane unit 3 when seen in the first flow direction A.

A storage chamber 40 is formed in the flow path 7 between the second opening(-s) 9 and the membrane unit 3, particularly the membrane 5, i.e. upstream of the membrane unit in the first flow direction A. the storage chamber 40 is a volume defined between the second opening(-s) 9 and if present the third opening(-s) 14 and the membrane unit 3.

The membrane unit 3 is accommodated in the nebulizer housing 17. The membrane unit 3 comprises a membrane 5 and an actuator 6. In the technical field given, such membrane units 3 are also known as "head units" or "heads".

The membrane 5 has a first side 5.1 (liquid side) and a second side 5.2 (aerosol side). The membrane 5 may be circular and may have a circular active area 62 in its center. The active area 62 is provided with a plurality of apertures 110. Further, the active area 62 may be domed towards the second side 5.2.

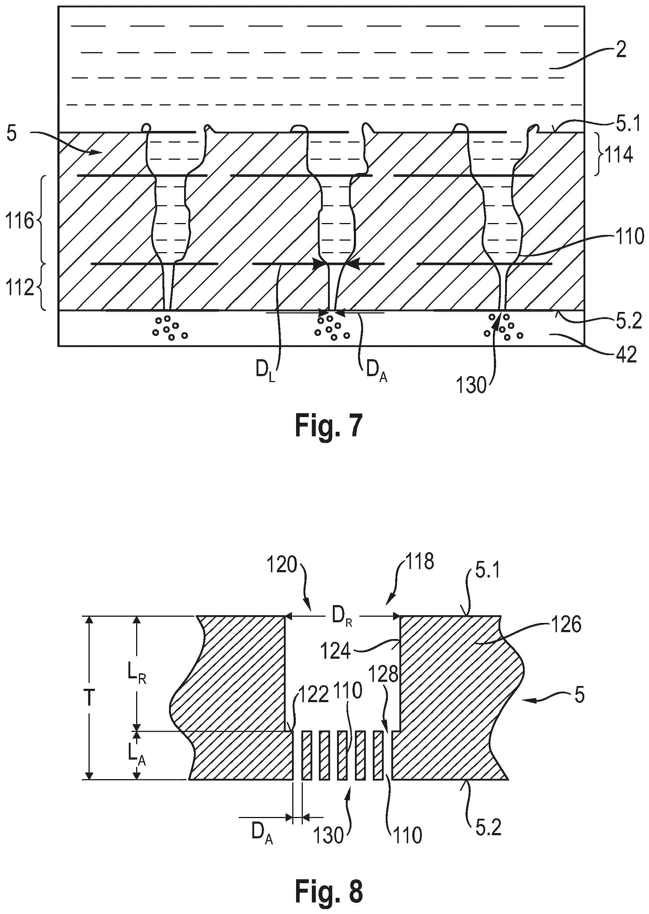

The apertures 110 may be drilled as shown in Figure 7. The apertures 110 penetrate the membrane 61 in the active area 62 from the liquid side 5.1 to the aerosol side 5.2. In use, the liquid solution 26 passes through the apertures 110 from the liquid side 5.1 to the aerosol side 5.2 when the membrane 61 is vibrated for generating the aerosol at the aerosol side 5.2 and emitting it into the mixing chamber 42. This aerosol may then be drawn by inhalation of a user from the mixing chamber 42 via the mouthpiece 27.

Figure 7 shows a cross-section (schematic CT picture) showing three of the apertures 110 of such a vibratable membrane 5. The through holes 110 of this particular embodiment are formed by laser drilling using three stages of different process parameters, respectively. In a first stage, the portion 114 is formed. In a second stage the portion 116 is formed and in a third stage the nozzle portion 112 is formed. In this embodiment, the average length of the nozzle portion 112 is 29.4µm, whereas the second stage portion 116 has an average length of 55.7µm. The first stage portion 114 has an average length of 16.3 µm. As a result, the total length of each aperture 110 is the sum of the length of the first portion 114, the second portion 116 and the nozzle portion 112, that is in this particular example 101.4 µm. Thus, the ratio of the total length of each aperture 110 to the length of a respective one of the nozzle portions 112 is approximately 4.3.

Alternatively, the apertures 110 may be laser milled and laser drilled as shown in Figure 8.

The vibratable membrane 5 in this example consists of a single layer 126 of unitary material, e.g. stainless steel. Yet, in other embodiments, the vibratable membrane 5 may comprise further layers attached to the liquid side 5.1 and/or the aerosol side 5.2. Additionally, other biocompatible metals may be used instead of stainless steel.

The exemplary vibratable membrane 5 comprises a plurality of recesses 118. The recesses 118 may be circular or annular.

The recesses 118 have an opening 120 at the liquid side 5.1 of the vibratable membrane 5. The recesses 118 are formed as blind holes having a bottom 122 opposite to the opening 120.

The recesses 118 have a circumferential side wall 124. In case of the circular recess 118, the circumferential side wall 124 corresponds to the sheath of a cylinder. Yet, in the cross-section in Figures 8, portions of the circumferential side wall 124 are opposite to (face) each other.

The recesses 118 may be formed in the single layer 128 by using an ultra-short-pulse laser. In this context, the recesses 118 are preferably made by laser milling, wherein the laser beam and/or the single layer 128 of the vibratable membrane 5 are translated relative to each other whereby material of the single layer 128 is gradually removed.

The smallest dimension D_{R} of the recesses 118 may be 300 µm. However, other dimensions are as well conceivable. For example, a smallest dimension D_{R} of the opening 120 of the recesses 118 facing the liquid side 5.1 may be between 40 µm and 500 pm, between 70 µm and 400 µm or between 90 µm and 300 µm.

In this context, the smallest dimension D_{R} for the circular central recess 118 corresponds to the diameter of the circle. The thickness T of the single layer 128 may be 100 µm.

The depth or length L_{R} of the recesses 118 may be selected between 80% to 95% of the thickness T of the single layer 128. Hence, the depth L_{R} of the recesses 10 may be between 80 µm to 95 µm.

However, the thickness T of the single layer 128 may reside in a range between 50pm to 200pm, optionally between 60pm and 150pm and more optionally between 70pm and 120pm. Additionally, the depth L_{R} of the recesses 10 may be selected to be equal to or more than 50% of the thickness T of the single layer 128.

Each recess 118 has a plurality of apertures 110 formed in the bottom 122 and extending from the bottom 122 to the aerosol side 5.2 of the single layer 128.

Each recess 118 may have at least 2, at least 20, or at least 50 or at least 100 of the apertures 110 formed in the bottom 122. At most each recess 118 may have 5,000 or 10,000 of the apertures 110.

Each aperture 110 has an entrance opening 128 at the bottom 122 and an exit opening 130 at the aerosol side 5.2 of the single layer 128. The apertures 110 may be substantially cylindrical or conical with the smaller opening being located at the aerosol side 9 and thus being the exit opening 130. Yet, the geometry is not limited in this regard and other geometries are as well conceivable.

The size of the exit openings 130 of the apertures 110 has a significant impact on the MMD. Depending on the fluid, the MMD is linearly larger than the diameter D_{A} of the exit openings 130 and, thus, directly proportional. Accordingly, the apertures 110 may be circular having a diameter D_{A} (diameter of the exit openings 130) facing the aerosol side 5.2 between 1 µm and 7 pm, preferably between 1.5 µm and 5 pm, and more preferred between 1.5 µm and 4.5 µm and most preferred between 1.5 µm and 3.5 µm. These values apply also to the embodiment in Figure 7.

A length L_{A} (which may also be referred to as height or depth) of the apertures 110 in the single layer 128 may be between 3 µm and 50 µm. In another example, the length L_{A} of the apertures 118 in the single layer 128 may be between 5 µm and 20 µm. In an even further example, the length L_{A} of the apertures 110 in the single layer 128 may be between 5 µm and 20 µm. Other ranges may be 5 to 16pm, 5 to 15pm, 5 to 12µm or 10 to 15µm. In this example of Figure 8, the apertures form the nozzle portion, particularly if formed by only one laser drilling stage. These values apply also to the embodiment in Figure 7.

Even further, the TOR and MMD are strongly dependent on the geometry of the nozzle portion 112, i.e. of the aperture 110 geometry (particularly length and diameter).

The apertures 110 may be formed by the same ultra-short-pulse laser used for forming the recesses 118. However, also a short-pulse laser, as used in the prior art, may be used for forming the apertures 110.

In any case, formation of the apertures 110 is preferably performed in one laser drilling stage at a substantially constant fluence of the laser. Only one drilling stage for forming the apertures is preferred to increase preciseness of the geometry of the apertures, i.e. the nozzle portion 112. When using just one laser drilling stage, the diameter at an entrance side (corresponding to the liquid side) and an exit side (corresponding to the aerosol side) will be very well defined with the above-described benefits.

In another aspect, the apertures 110 in the bottom of the recesses 118 may also be drilled with two laser drilling stages having a different fluence similar to the embodiment above using the three drilling stages. For example a first laser drilling stage with a first fluence may be used to form a first part of the aperture in the bottom of the recess and a second laser drilling stage with a second fluence lower than the first fluence may be used to finalize the aperture, i.e. completely penetrate the bottom. In this instance, the nozzle portion may be defined by the second laser drilling stage only. Thus, the bottom may remain thicker (lower depth of the recess) when using more than one laser drilling stage for forming the apertures. This may provide for more mechanical rigidity of the aperture plate.

The method of manufacturing may include as a first step the formation of the recesses 118 from the liquid side 5.1 of the single layer 128.

In a second, subsequent step, the apertures 110 are formed in the bottoms 122 of the recesses 118 from the liquid side 5.1 of the single layer 128.

Subsequently, the vibratable membrane 5 may be domed.

The membrane 5 may be attached to a support. Yet, in the present embodiment, the membrane 5 has an annular portion about its active area 62. The membrane 5 further comprises an annular ring 29 concentric to the membrane 5, particularly its annular portion and active area 62. The annular ring 29 is connected to the membrane 5 via a plurality of spokes 32. Moreover, the membrane 5 has an annular portion about its active area 62. The actuator 6 being directed coupled, that is attached to the annular portion about the active area 62 of the membrane 5.

The actuator 6 is electrically connected to a plug 33 of the membrane unit 3 for electrical connection to the later described controller 18.

As will be apparent particularly from figure 3, the membrane unit 3 is arranged within the nebulizer housing 17 so as to close a supply opening 34 of the reservoir 2 located at the bottom of the reservoir 2. To be more precise, the membrane 5 closes with its first side 5.1 the supply opening 34. To provide a seal, a sealing lip 35 is provided about the circumference of the supply opening 34 (see figure 4). The membrane 5 abuts the sealing lip 35 with its first side so that the active area 62 of the membrane 5 is surrounded by the sealing lip 35. As a consequence, liquid filled into the reservoir 2 will by gravitational force be fed to the supply opening and, hence, to the first side 5.1 of the membrane 5.

To allow replacement and/or cleaning of the membrane unit 3, the membrane unit 3 is interchangeable. In an embodiment, the nebulizer housing 17 comprises a main body 30 and a door 31. The circumferential wall 10 is formed by the main body 30 and to the door 31. The door 31 is hinged to the main body 30. The door 31 may, thus, be moved between an open and a closed position. In the open position (see figure 4) the membrane unit 3 may be inserted into a head mount 36 so that the circumference of the active area 62 at the first side 5.1 of the membrane 5 abuts the sealing lip 35. In order to prevent the membrane unit 3 from being incorrectly mounted, the head mount 36 comprises a key recess 37 into which a key element 38 of the membrane unit 3 is to be inserted during mounting. Upon closing of the door 31, the membrane unit 3 is pressed towards the main body 30, whereby the first side 5.1 of the membrane 5 is pressed against the sealing lip 35. In the closed position, the door 31 is fixed to the main body 30 by using the latch 5.

When placing the membrane unit 3 inside the flow path, a cavity is formed between the membrane unit 3 and the second opening(-s) 9 which would be hard to clean. By configuring the nebulizer housing 17 to comprise the main body 30 and the hinged door 31, the membrane unit 3 may be removed and the cavity is accessible for being cleaned. Yet, providing the latch 18 and configuring the nebulizer housing 17 to comprise the main body 30 and the hinged door 31 is not compulsory. It is also possible to provide a nebulizer housing 17 that can be opened differently by e.g. configuring the main body 30 and the door 31 separately and/or provide some kind of fastening device to fasten them together, such as a magnet, a latch, a press-fitting, a zipper, a press button and the like. Further, it is also possible to provide a nebulizer housing 17 that cannot be opened by a user, operator, hospital staff or the user itself. This is may, for example, be achievable by forming a nebulizer housing 17 in which the membrane unit 3 is arranged in a fixed position and cannot be replaced. In this instance, it may even be conceivable that the whole nebulizer housing 17 is a disposable and will be disposed from time to time.

As will be best apparent from figure 3, an angle α₄ between the support surface 19 and the membrane 5 is, in side view (longitudinal cross-sectional view), more than 90°±5°, optionally between 100° and 160° and more optionally between 110 and 150°.

Taking the aforesaid into account, the nebulizer housing 17 defines the mouthpiece 27, accommodates the membrane unit 3 and forms the flow path 7 between the first opening 8 and the second opening(-s) 9.

As will be apparent from figure 3, the membrane 5 of the membrane unit 3 is disposed in the flow path 7. In addition, the flow will, during inhalation, enter the housing body 4, particularly the nebulizer housing 17 via the second opening(-s) 9 flowing into the storage chamber 40 formed between the second opening(-s) 9 and the membrane 5 of the membrane unit 3. Moreover, flow path openings 39 (see figure 4) are formed in a wall 41 comprising the head mount 36 so that air may flow from the storage chamber 40 through the flow path openings 39 and the open spaces between the spokes 32 of the membrane 5 into the mixing chamber 42. Accordingly, an envelope flow about at least part of the circumference of the membrane 5 or more particularly in the present embodiment about the circular disc having the active area 62 of the membrane 5 is formed. To put it differently, the membrane unit 3 may be arranged in the flow path 7, such that a sheathing or enveloping flow can pass around the membrane unit 3 inside the flow path 7 during inhalation, and during exhalation, of the user during breathing through the inhalation therapy device 1. In an embodiment, the membrane unit 3 may be arranged substantially (±10° or ±5°) in an axially centred position. In particular, it may be useful that a plane normal of the membrane is as parallel as possible to the mouthpiece axis CA₁). In order to deposit as little aerosol as possible in the device, direct nebulization in the direction of the first opening 8 is desired. For this purpose, a (nearly) perpendicular position of the membrane 5 to the mouthpiece axis CA₁ is desired. The plane normal of the membrane (CA₃) should therefore not intersect with the inner walls of the flow path 7.

Aerosol is generated into the mixing chamber 42 upon operation of the actuator 6 on the second side 5.2 of the membrane 5 upon inhalation of a user or during idling without a user breathing through the device. Hence, the envelope flow generated upon inhalation entrains the generated aerosol for being output through the first opening 8, being the outlet opening of the mouthpiece 27 for being administered/supplied to a user.

Upon exhalation of a user, exhaled air will enter the flow path 7 via the first opening 8 at the mouthpiece 27 and flow via the mixing chamber 42 and the open spaces provided about the circumference of the membrane 5 between the spokes 32 and the flow path openings 39 into the storage chamber 40. The exhaled air may then exit the flow path 7 via the second opening(-s) 9 and the third opening(-s) 14. Upon exhalation, that is in the second flow direction B, the check valve 15 is configured to open and let air pass through the third opening(-s) 14 and being expelled to the outside of the flow path 7 and the nebulizer housing 17. To the contrary, the check valve 15 closes the third opening(-s) 14 upon inhalation, that is in the first flow direction A. As a result, air may substantially exclusively enter the flow path 7 via the second opening(-s) 9 upon inhalation of a user and air may substantially exclusively exit the flow path 7 via the second opening(-s) 9 and the third opening(-s) 14 upon exhalation of a user.

To evaluate the quality of the inhalation therapy and to deliver a signal for controlling the actuator, it is required to observe the way the inhalation and the exhalation through the inhalation therapy device 1 is performed. In this connection a "good therapy" equals a therapy, in which a high amount of drug is delivered to the user in a short time. This can be achieved by limiting the inhalation flow and extended inhalation durations at shortened exhalation durations without overstraining the user's inhalation behaviour. In such a configuration, an unpleasant feeling for the user can be avoided, which could increase the chances that the therapy is interrupted by the user.

Hitherto, the preferred embodiments of the present disclosure found an easy to manufacture and disinfect solution that achieves a beneficial short overall therapy time, in which the inhalation time can be prolonged, and the exhalation time can be kept as short as possible and without unpleasant resistances during the exhalation.

To achieve the prolonged inhalation time, it is required that the user at least inhales against a certain resistance, i.e., a certain flow resistance. The above configuration allows to achieve the desired inhalation flow resistance and, at the same time, allows to reduce the exhalation flow resistance, as the check valve 15 facilitates the expel of air via the check valve 15 and the third opening(-s) 14 of the flow path 7 upon exhalation.

In an alternative embodiment, the third opening(-s) and the check valve 15 may also be omitted. In such an embodiment, air may upon inhalation exclusively or substantially exclusively enter the flow path 7 via the second opening(-s) 9 and air may upon exhalation exclusively or substantially exclusively be expelled from the flow path 7 via the second opening(-s) 9. This is hence a scenario, in which the entire flow volume of the inhalation and the exhalation are guided through the second opening(-s) 9 of the flow path 7. The increased inhalation resistance and/or reduced exhalation resistance in this configuration is created using a fixed geometry valve functioning as the second opening.

Turning now to the controller housing 16 in more detail. The controller housing 16 accommodates the controller 18 configured to control the actuator 6 of the membrane unit 3.

A top surface/upper surface (curved surface) 21 of the controller housing 16, in the handle portion 12, is rounded or curved providing for a good ergonomic handling of the controller housing/handle portion 16. The top surface/upper surface 21 is located opposite to the bottom surface of the controller housing 16 defining the support surface 19. The top surface 21 defines in a side view (see figure 2) a ridge line 22. In this context, a ridge line is to be understood as a line formed along the highest points of the upper surface in side view (longitudinal cross-sectional view). An angle α₆ between the ridge line 22 and the support surface 19 is, in side view (longitudinal cross-sectional view), less than 20°, optionally between 5° and 15°.

At least one control button 23 and/or at least one light indicator 24 are located on the top surface 21, here centered on the ridge line 22. A user may switch the aerosol therapy device ON and OFF by means of the control button 23. The light indicator 24 may provide feedback to the user relating to the progress of the treatment.

In the present embodiment, the nebulizer housing 17 is detachable from the controller housing 16. In this context, the nebulizer housing 17 comprises a circumferential rim 43 defining a recess 44. The controller housing 16 comprises a boss 45, wherein the recess 44 and the boss 45 are engaged, when the nebulizer housing 16 of the controller housing 16 are assembled.

Further, the controller housing 16 comprises a socket 46. Upon mounting of the nebulizer housing 17 into the controller housing 16, the plug 33 of the membrane unit 3 may releasably be plugged into the socket 46, whereby the membrane unit 3 is electrically connected to the controller 18 via the socket 46.

In an example, an angle α₅ between the support surface 19 and a plugging direction of the plug 33 into the socket 46 is, in side view (longitudinal cross-sectional view), more than 90°±5°, optionally between 100° and 160° and more optionally between 110 and 150°.

Further, a sensor 47 is accommodated in the controller housing 16. The sensor 47 is electrically connected to the controller 18. In the present example, the sensor 47 is a sensor for detecting a differential pressure between ambient pressure and a pressure in the flow path, here between the second opening(-s) at 9 and the membrane 5, that is in the present embodiment the storage chamber 40. For this purpose, the sensor 47 has a sensor port 48. The sensor port 48 is in the present embodiment located in the bottom wall 51 of the nebulizer housing 17. A tube or hose 49 connects the sensor port 48 with the sensor 47. The ambient pressure is in this configuration taken from the interior of the controller housing 16, which is not hermetically sealed so that the pressure within the controller housing 16 corresponds to ambient pressure.

Furthermore, the controller housing comprises and accommodates a battery or batteries. The battery or batteries may be rechargeable and/or exchangeable. The battery/-ies is/are disposed in proximity of the support surface 19 and extending with its/their length/-s along the support surface 19. In this context, a battery is to be understood as any container consisting of one or more cells, in which chemical energy is converted into electricity and used as a source of power. Further, along the support surface does not necessarily mean extending parallel to the support surface. Further, along the support surface 19 does not necessarily mean that they extend parallel to the support surface 19. Yet, the longitudinal direction of the battery/-ies should be within 0° and 20° and preferred within 0° and 15° relative to the support surface 19.

Taking the aforesaid into account, the controller housing 16 may comprise the most relevant, preferably all, electronic features necessary to operate the membrane unit 3 and other features of the inhalation therapy device 1 such that the nebulizer housing 17 can be configured as a throw-away (disposable) unit or a unit, which is to be used for a certain amount of time, such as a certain number of therapies, weeks, months, half-year or maximal one year. At the same time, the controller housing 16 comprising all relevant electric and sensory elements for operating the membrane unit 3 and other features of the inhalation therapy device and monitoring the therapy process may be construed as a long-lasting unit, which does not need to be specifically disinfected before every therapy session.

The embodiment illustrated in the figures is an inhalation therapy device 1 through which a user inhales and exhales during the therapy. That is, the inhalation therapy device 1 is brought into contact with the user's mouth such that the regular inhalation and exhalation of the user is performed vie the first opening 8 through the inhalation therapy device 1. In an alternative embodiment, a mask or a mouthpiece may even be attached to the first opening 8. In either case, the inhalation therapy device 1, specifically its first opening 8 shall not be removed from the user's mouth (or face if a mask is used) during the treatment. Yet, in case the patient removes the inhalation therapy device 1, specifically its first opening 8, from the user's mouth (or face if a mask is used) during the treatment, the membrane unit 3 is simply deactivated and stops aerosolizing. Hence, breaks are possible, though not intended.

How the aerosol is generated and how the actual therapy is to be performed inside the inhalation therapy device 1 will be described in the following.

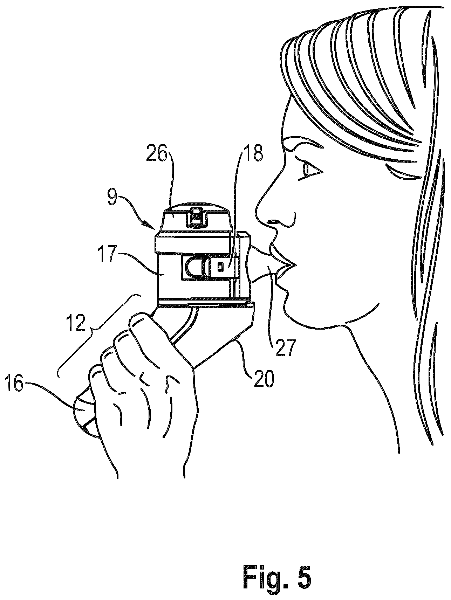

Here and in the following, it shall be understood that Figure 5 shows the orientation of the inhalation therapy device when it is held by a user in an ideal position during therapy in a standing or sitting position of the user.

In this scenario, a reservoir 2 for holding a liquid or fluid (i.e., the medicament) is provided at an upper section of the housing body 4 with the central axis CA₂ of the filling opening 25 being substantially vertical.

Additionally, it is derivable from Figure 5 that also the membrane unit 3 is oriented vertically. Thus, the center axis CA₃ of the membrane 5 is oriented horizontally. Accordingly, any liquid in the reservoir 2 is supplied to the membrane 5 by gravitational force.

As mentioned earlier, it is the first opening 8 that is to be brought into contact with the user's respiratory tract via his or her mouth to enable inhalation and exhalation through the inhalation therapy device during the aerosol therapy.

In use, when a user wants to start a therapy, the first step is to completely assemble the inhalation therapy device 1 including inserting the membrane unit 3 into the nebulizer housing 17, closing the nebulizer housing 17 and attaching the nebulizer housing 17 to the controller housing 16. Moreover, the lid 26 has to be unscrewed from the reservoir 2 and the to be administered liquid has to be poured into the reservoir 2 via the filling opening 25. when the inhalation therapy device 1 is placed with its support surface 19 on a horizontal surface such as a table, a plane of the filling opening 25 will be angled towards the mouthpiece 27 relative to the horizontal surface. Yet, pouring liquid into the reservoir 2 via the filling opening 25 is still enabled without liquid flowing out of the reservoir 2. Subsequently, the lid 26 will again be screwed to the reservoir 2 to seal the liquid. In a final step, the user switches the inhalation therapy device 1 ON by a pressing the control button 23. At this stage, the user may start the treatment.

To start a treatment, the user/user will put the mouthpiece 27 in his/her mouth and start inhaling and exhaling. In this context, one breathing cycle consists of only one inhalation phase and only one subsequent exhalation phase.

When the user starts inhaling, the user will inhale against the maximum absolute value of the first flow resistance of about 160 Pa ± 30 Pa optionally 160Pa ± 60Pa, more optionally 160Pa ± 30Pa, measured at a peak flow of a sinus-shaped breathing pattern of two seconds of inhalation time and two seconds of exhalation time, respectively, and 500 ml of tidal volume. In an embodiment, the maximum absolute value of the first flow resistance may be between 120 Pa to 500 Pa, optionally between 125 Pa to 400 Pa, even more optionally between 130 Pa to 300 Pa, and most optionally between 135 Pa to 180 Pa. In a further embodiment, the maximum absolute value of the first flow resistance upon inhalation of the user may be between 60Pa and 260Pa, preferably 100Pa and 220Pa, more preferably 130Pa and 190Pa.

In the course of the inhalation, an inhalation flow A, as described above, will be induced in the flow path 7. In the current embodiment one pressure port (or joint) of the differential pressure sensor in the controller is in fluidic communication with the flow path 7 via the sensor port 48. The other pressure port (or joint) of the differential pressure sensor in the controller is in fluidic communication with the environment such, that the differential pressure sensor 47 can measure the pressure difference between the environment (ambient pressure) and the inside of the nebulizing unit. Upon inhalation, the pressure in the flow path will be a negative pressure.

The controller 18 may, based on this output from the sensor 47, conclude on inhalation of a user. Upon conclusion on inhalation or the end of exhalation (in the case of active pre-on), the controller 18 activates the actuator 6, whereby the membrane 5 is vibrated and aerosol is generated from the liquid in the reservoir 2 on the second side 5.2 of the membrane 5. Due to the inhalation flow A, the generated aerosol will be inhaled by the user and be transported to the desired location within the respiratory tract.

At the end of the first inhalation phase, the controller 18 concludes from the pressure signal inside the flow path that the inhalation phase is close to an end, therefore deactivates the actuator 6 and, thereby, stops the aerosol production. In a particular example, the controller 18 will therefore before the inhalation phase ends deactivate the actuator 6 and, thereby, stop the aerosol production (Pre-Off process). Accordingly, at the end of the inhalation phase, the user will no longer inhale aerosol. The timing in this regard is preferably adapted to allow that all aerosol reaches the desired location (the lung) and will not remain unused in the trachea and throat (respiratory dead volume) and will be exhaled at time exhalation starts, maximizing the dose delivered to the user's lung. The time period during which the actuator 6 is stopped at the end of the inhalation phase may be between 100 ms and 1000 ms, optionally 300 ms to 800 ms, most optionally 50 ms before the exhalation phase starts, related to the standard sinus-shaped breathing pattern of two seconds of inhalation time and two seconds of exhalation time, respectively, and 500 ml of tidal volume. As an alternative to the time, the controller may conclude on an exhalation target volume (target respiratory dead volume) of 150ml, optionally 130ml and more optionally 100ml. Due to the Pre-Off process, all or at least most of the aerosol will reach the desired location (the lung) within the respiratory tract and only a small amount of aerosol will again be exhaled in the consecutive exhalation phase.

In the consecutive exhalation phase, the user exhales producing the exhalation flow B in the flow path against a maximum absolute value of a second flow resistance of approximately 120 Pa (± 75 Pa) measured at the peak flow of the sinus-shaped breathing pattern of two seconds of inhalation time and two seconds of exhalation time, respectively, and 500 ml of tidal volume and optionally a maximum absolute value of the second flow resistance of approximately 120 Pa (± 45 Pa) and most preferred of 120 Pa (± 22.5 Pa).

When starting exhalation, the pressure (overpressure) within the flow path 7 again changes to overpressure and the controller 18 may, based on the output from the sensor 47, conclude on exhalation of a user. At the end of the exhalation phase, the pressure within the flow path 7 will be reduced, approaching ambient pressure allowing the controller 18 to conclude that the exhalation phase is close to an end. In a particular example, the controller 18 will therefore before the exhalation phase ends activate the actuator 6 and, thereby, start the aerosol production (Pre-On process). The time period during which the actuator is operated during the exhalation phase may be between 50 ms and 500 ms, optionally 100 ms to 300 ms, most optionally 200 ms, related to a standard sinus-shaped breathing pattern of two seconds of inhalation time and two seconds of exhalation time, respectively, and 500 ml of tidal volume. Alternatively, time period during which the actuator is operated during the exhalation phase may be up to 500ms, up to 300ms, up to 200ms or up to 100ms.

As the aerosol production is already started during the exhalation phase and when an exhalation flow B is still present, some of the produced aerosol will be transported to a position downstream of the membrane 5 in a direction of the second opening(-s) 9 and the optional third opening(-s) 14. In order to prevent that produced aerosol during this time period is flown out of the inhalation therapy device 1, that is through the second opening(-s) 9 and the optional third opening(-s) 14, the storage chamber 40 is provided. In other words, the produced aerosol is flown during this time period into the storage chamber 40 to temporarily store the aerosol as a bolus until the next consecutive inhalation phase starts.

Subsequently, the user will start the next breathing cycle with the next inhalation phase. At the beginning of inhalation, the aerosol temporarily stored in the storage chamber 40 will again be entrained by the inhalation flow A, past the membrane unit 3 and be inhaled via the first opening 8. Upon starting with next inhalation phase, the pressure in the storage chamber 40 will again change allowing the controller 18 to conclude on the next inhalation phase. Thus, the controller 18 will again activate the actuator 6 starting with the aerosol production. At this stage, the above-described process is repeatedly performed.

It is to be mentioned, that aerosol production may be continuous between the process Pre-On and Pre-Off (phase of operation). Yet, the power supplied to the actuator 6 (power adaption), the frequency (frequency adaption), etc. may change between consecutive phases of operation or even within one phase of operation.

If no liquid is left in the reservoir 2 or if the amount of liquid remaining in the reservoir has reached the minimum needed to produce aerosol, end of dose is reached, and the process (therapy) is stopped. End of dose may be detected by the inhalation therapy device 1 and be indicated to the user visually using the light indicator 24 or a light integrated into the control button 23 and/or auditory by outputting an audio signal. In this regard, end of does can be detected as described in EP 1 558 315 B1 (US 7,458,372 B2) or EP 3 082 918 B1 (US 10,744,277 B2).

Finally, the user may switch the inhalation therapy device again OFF using the controller bottom 23 to conclude the therapy. Alternatively, the device may automatically switch of after end of dose has reached and a certain amount of time has lapsed since then.

## Claims

1. Inhalation therapy device comprising:
a housing (4) having
a handle portion (12) for holding the inhalation therapy device during inhalation
a support surface (19) for placing the inhalation therapy device on a horizontal surface and
a reservoir (2) for holding a liquid to be nebulized;
a membrane unit (3) disposed in the housing and having
a membrane (5) comprising a plurality of apertures (110), wherein the membrane (5) is disposed so that, when liquid is held in the reservoir (2), the liquid is supplied to a first side (5.1) of the membrane (5);
an actuator (6) coupled to the membrane (5) for vibrating the membrane (5), whereby the liquid passes through the apertures (110) and an aerosol is generated at a second side (5.2) of the membrane (5) opposite to the first side (5.1); and
a mouthpiece (27) having an outlet opening (8) defined by an outer edge (28) of the mouthpiece (27) for being put into the mouth of a user for inhaling the aerosol,
wherein a center axis (CA₁) of the outlet opening (8) is non-parallel to the support surface (19) so that the outer edge (28) of the mouthpiece (27) is oriented in a direction towards the support surface (19) without interfering with a virtual extension (13) of the support surface (19).

2. Inhalation therapy device according to claim 1, wherein the minimum distance (D) between the virtual extension (13) of the support surface (19) and the outer edge (28) of the mouthpiece (27) in a direction perpendicular to the virtual extension (13) of the support surface (19) is more than 3 mm and less than 15 mm.

3. Inhalation therapy device according to claim 1 or 2, wherein the center axis (CA₁) of the outlet opening (8) intersects the virtual extension (13) of the support surface (19) on a mouthpiece side with respect to the support surface and an angle (α₁) between the virtual extension (13) of the support surface (19) and the center axis (CA₁) of the outlet opening (8) is, in side view, an acute angle of less than 75°, optionally between 10° and 70°, optionally between 15° and 60°, optionally between 20° and 50°.

4. Inhalation therapy device according to any one of the preceding claims, wherein the reservoir (2) has a filling opening (25) for receiving the liquid and a lid (26) for closing the filling opening (25), wherein an angle (α₂) between the support surface (19) and a center axis (CA₂) of the filling opening is, in side view, an obtuse angle of more than 90°±5°, optionally between 100° and 160° and more optionally between 110 and 150°.

5. Inhalation therapy device according to claim 4, wherein an angle (α₃) between the center axis (CA₂) of the filling opening and the center axis (CA₁) of the mouthpiece (27) is, in side view, between 70° and 110° and optionally between 80° and 100° and even more optionally 90°±5°

6. Inhalation therapy device according to any one of the preceding claims, wherein an angle (α4) between the support surface (19) and the membrane (5) is, in side view, more than 90°±5°, optionally between 100° and 160° and preferred 110° and 150°.

7. Inhalation therapy device according to any one of the preceding claims, wherein the handle portion (12) comprises the support surface (19) and a curved surface (21) opposite to the support surface (19) wherein the curved surface (21), in side view, defines a ridge line (22).

8. Inhalation therapy device according to claim 7, wherein, an angle (α₆) between the ridge line (22) and the support surface (19) is, in side view, less than 20°, optionally between 5° and 15°.

9. Inhalation therapy device according to claim 7 or 8, further comprising at least one control button (23) and/or at least one light indicator (24) located on the curved surface (21), optionally centered on the ridge line (22).

10. Inhalation therapy device according to any one of the preceding claims, wherein the housing (4) comprises
a nebulizer housing (17) having the reservoir (2) and the mouthpiece (27) and accommodating the membrane unit (3), and
a controller (16) housing accommodating a controller (18) configured to control the actuator (6) of the membrane unit (3) and having the support surface (19) and the handle portion (12),
wherein the nebulizer housing (17) is detachable from the controller housing (16).

11. Inhalation therapy device according to claim 10, wherein one of the nebulizer housing (17) and the controller housing (16) comprises a circumferential rim (43) and the other of the nebulizer housing (17) and the controller housing (16) comprises a boss (45), wherein the circumferential rim (43) and the boss (45) are engaged.

12. Inhalation therapy device according to claim 10 or 11, wherein the nebulizer housing (17) defines a flow path (7) from at least one inlet opening (9) of the nebulizer housing (17) to the outlet opening (8) of the mouthpiece (27), wherein a flow is generatable in the flow path (7) upon inhalation of a user from the inlet opening (9) passed the membrane unit (3) to the outlet opening (8) for entraining and delivering the generated aerosol and/or upon exhalation of a user from the outlet opening (8) passed the membrane unit (3) to the inlet opening (9).

13. Inhalation therapy device according to claim 10, 11 or 12, wherein the controller housing (16) comprises a socket (46) and the membrane unit (3) comprises a plug (33) releasably plugged into the socket (46) in a plugging direction, wherein an angle (α₅) between the support surface (19) and the plugging direction is, in side view, more than 90°±5°, optionally between 100° and 160° and optionally between 110° and 150°.

14. Inhalation therapy device according to any one of the preceding claims, further comprising at least one battery (50) accommodated in the housing (4), the battery (50) being disposed in proximity of the support surface (19) and optionally extending with its longitudinal extension along the support surface (19).

15. Inhalation therapy device according to any one of the preceding claims, wherein the housing (4) comprises feet (20) and the support surface (19) is a level surface formed by the respective stand surfaces of the feet (20).
